# FASCICULE DE BREVET EUROPEEN

(11) **EP 1 400 260 B1**
(45) Date de publication et mention de la délivrance du brevet: **14.06.2006**
(21) Numéro de dépôt: 03292172.8
(22) Date de dépôt: 04.09.2003
(51) Int. Cl.: A61N 1/39, A61N 1/362

(54) **Dispositif implantable actif du type défibrillateur, cardioverteur et/ou stimulateur antitachycardique, à discrimination perfectionnée entre tachycardie et fibrillation ventriculaires**
Implantierbare aktive Vorrichtung Defibrillator, Kardioverter und/oder Herzschrittmacher zur Behandlung von Tachykardien, mit verbesserter Diskriminierung zwischen Tachykardie und ventrikulärer Fibrillation
Implantable active device like defibrillator, cardioverter and/or antitachycardia pacing, including an improved discriminating between tachycardia and ventricular fibrillation

(30) Priorité: 04.09.2002 FR 0210912
(43) Date de publication de la demande: 24.03.2004
(73) Titulaire: ELA MEDICAL, 92541 Montrouge (FR)
(72) Inventeur: Henry Christine, 75014 Paris (FR)
(74) Mandataire: Dupuis-Latour, Dominique

(56) Documents cités:
- EP-A- 0 838 235
- EP-A- 1 129 746

## Description

La présente invention concerne les dispositifs médicaux implantables actifs (au sens de la directive 90/385/CEE du 20 juin 1990 du Conseil des communautés européennes), et plus particulièrement les appareils communément appelés "défibrillateurs implantables" ou "appareils de cardioversion", étant entendu que l'invention couvre aussi bien les défibrillateurs/cardioverteurs/stimulateurs implantables que les défibrillateurs/stimulateurs implantables)

Ces appareils sont chargés de diagnostiquer certaines tachyarythmies et de délivrer au coeur des "chocs" de défibrillation ou de cardioversion (impulsions électriques de haute énergie, dépassant notablement l'énergie fournie pour la simple stimulation).

Certains d'entre eux incorporent également un mode de thérapie dit "ATP" (*AntiTachycardia Pacing*) agissant par stimulation programmée à haute fréquence.

La décision d'appliquer une thérapie antitachycardique, et le choix de cette thérapie (choc ou stimulation ATP) est prise par un algorithme de détection et de classification des différentes tachyarythmies en fonction de plusieurs critères, principalement en fonction de la fréquence ventriculaire, mais également en fonction de la stabilité des intervalles ventriculaires, de la stabilité de conduction auriculo-ventriculaire, du mode de démarrage des tachycardies, etc. (voir notamment les EP-A-0 626 182 et EP-A-0 838 235 au nom de ELA Médical).

Si l'on met à part les tachyarythmies qui ne sont pas justiciables de thérapies ventriculaires - notamment les tachycardies sinusales (TS) ou tachycardies supra-ventriculaires (TSV), qui sont d'origine auriculaire et pour lesquelles un choc appliqué au ventricule serait inefficace, voire délétère -, il importe que l'algorithme opère également une discrimination entre une tachycardie ventriculaire (TV) rapide et une fibrillation ventriculaire (FV).

En effet, ces deux formes de tachycardie doivent être traitées de manière différente :
- en cas de fibrillation ventriculaire avérée ou de TV polymorphe rapide instable, il est important d'appliquer le plus rapidement possible une thérapie de choc, seule susceptible de faire cesser cette fibrillation ;
- en revanche, en cas de tachycardie ventriculaire monomorphe rapide ou lente, il n'est pas a priori systématiquement nécessaire ni utile d'appliquer un choc ; une stimulation ATP peut être appliquée en première intention, qui peut être efficace et éviter l'application d'un choc, plus douloureux.

Les algorithmes de classification des tachyarythmies opèrent en fonction de plusieurs critères, dont le premier est celui de la fréquence ventriculaire ; si une analyse plus fine est nécessaire, l'algorithme évalue en outre la stabilité des intervalles RR (intervalles ventriculaires), la stabilité des intervalles PR (association auriculo-ventriculaire), la présence ou non d'une accélération brusque du rythme et l'origine, ventriculaire ou auriculaire de cette accélération.

Le premier critère, la fréquence ventriculaire, permet en particulier de distinguer trois situations, selon que cette fréquence est plus ou moins élevée (ou, de façon équivalente, que l'intervalle RR est plus ou moins court).

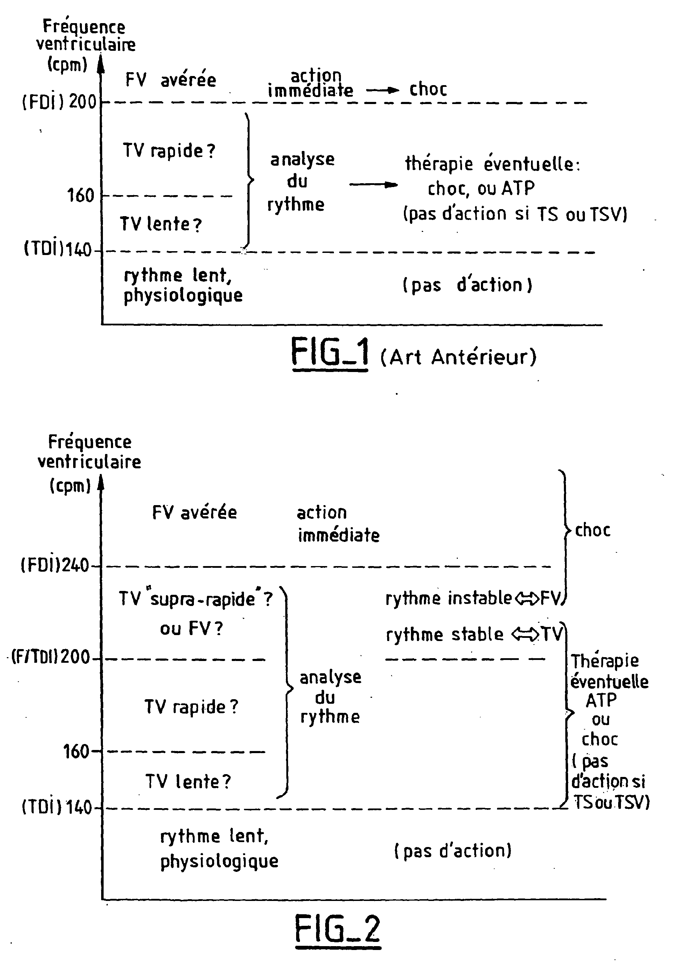

Ces situations sont illustrées sur la figure 1, qui représente les différentes plages de fréquence ventriculaire avec les diagnostics et actions correspondants :
- fréquence inférieure à un seuil donné, dit "fréquence de détection de TV" ou "seuil TDI" (*Tachycardia Detection Interval*), par exemple de l'ordre de 140 cpm : l'algorithme considère que ce rythme, lent, n'est pas pathologique et ne justifie jamais l'application d'une thérapie ;
- fréquence comprise entre la fréquence de détection de TV, typiquement 140 cpm, et un autre seuil, de fréquence supérieure, dit "fréquence de détection de FV" ou "seuil FDI" (*Fibrillation Detection Interval*), typiquement 200 cpm : l'algorithme considère alors qu'il y a "suspicion de TV" et opère une analyse plus approfondie, mettant en oeuvre d'autres critères que la fréquence ventriculaire, pour déterminer plus précisément le type de trouble et décider s'il y a lieu ou non d'appliquer une thérapie, et quel type de thérapie (choc ou stimulation ATP) ;
- fréquence supérieure à la fréquence de détection de FV, typiquement 200 cpm : l'algorithme considère que l'application d'une thérapie est en tout état de cause nécessaire, et sans délai.

Une difficulté réside dans le choix du niveau auquel doivent être fixés ces seuils, notamment le seuil FDI qui est un seuil critique, car il permet de discriminer les FV des TV.

Cette valeur de seuil étant paramétrable par le praticien, la tendance des implanteurs est de programmer le seuil de détection des FV (seuil FDI) à une valeur relativement élevée, typiquement supérieure à 220 cpm, afin que la majorité des TV puissent bénéficier de la stimulation ATP, qui n'est programmable que dans la zone de TV. L'efficacité de l'ATP sur des TV monomorphes rapides (220 à 240 cpm) est en effet bien documentée et, comme l'ATP est beaucoup moins douloureux pour le patient, il paraît préférable de traiter ces pathologies par stimulation ATP plutôt que par application d'un choc.

Mais la détection des FV peut se trouver perturbée par la fixation de ce seuil FDI à un niveau relativement élevé.

En effet, en cas de FV le signal cardiaque est instable et son couplage peut parfois être plus court que la durée de la période réfractaire absolue du dispositif, ce qui conduit à détecter ce rythme en 2:1 ; par ailleurs, certaines ondes sont de faible amplitude et peuvent être sous-détectées.

Dans ces conditions, et tel que fonctionne l'algorithme de détection des tachycardies dans les défibrillateurs tels que ceux décrits dans les documents précités, il peut suffire de trois cycles sur huit présentant un intervalle de couplage de durée supérieure à l'intervalle de détection de fibrillation (intervalle FDI) pour que la FV soit vue en zone de tachycardie, conduisant ainsi à un faux négatif dans le diagnostic. Cette tachycardie étant instable, l'algorithme porte un diagnostic de TS ou TSV (du fait de l'instabilité des intervalles RR), conduisant à une inhibition de la thérapie et donc à un retard dans l'application du choc de défibrillation.

L'un des buts de l'invention est de pallier cette difficulté, en proposant un dispositif qui autorise la programmation d'un seuil de détection des fibrillations ventriculaires à un niveau élevé, tout en permettant une discrimination exacte et sans délai entre FV et TV rapides susceptibles de se produire dans cette zone de fréquences. Le traitement pourra être ainsi adapté au mieux : délivrance immédiate d'un choc en cas de FV, thérapie sans choc par stimulation ATP en cas de TV rapide.

À cet effet, l'invention propose un défibrillateur ou cardioverteur de type en lui-même connu tel que décrit par exemple par le EP-A-0 838 235 précité, c'est-à-dire comprenant : des moyens de recueil de l'activité cardiaque ventriculaire et auriculaire ; des moyens pour appliquer une thérapie antitachycardique par choc de défibrillation ou de cardioversion et/ou par stimulation antitachycardique ; et des moyens discriminateurs d'arythmies ventriculaires, aptes à analyser le rythme ventriculaire recueilli par rapport à une pluralité de critères incluant la fréquence ventriculaire, qui est comparée à une pluralité de seuils comprenant un premier seuil de détection des tachycardies ventriculaires et un deuxième seuil de détection des fibrillations ventriculaires. Les moyens discriminateurs inhibent l'application des thérapies de choc et de stimulation antitachycardique lorsque la fréquence est inférieure au premier seuil, suspectent une tachycardie ventriculaire et poursuivent l'analyse du rythme sur lesdits critères lorsque la fréquence est comprise entre le premier seuil et le deuxième seuil, et commandent l'application d'une thérapie de choc lorsque la fréquence est supérieure au deuxième seuil.

De façon caractéristique de l'invention, les moyens discriminateurs sont également aptes à comparer la fréquence à un troisième seuil compris entre le premier seuil et le deuxième seuil, et à suspecter une tachycardie ventriculaire et poursuivre l'analyse du rythme sur lesdits critères seulement lorsque la fréquence est comprise entre le premier seuil et le troisième seuil, et opérer une discrimination additionnelle entre tachycardie ventriculaire et fibrillation ventriculaire lorsque la fréquence est comprise entre le troisième seuil et le deuxième seuil.

Ce troisième seuil est typiquement compris entre 190 et 210 cpm, pour un deuxième seuil compris entre 230 et 250 cpm, et/ou avec un intervalle entre ces seuils compris entre 20 et 50 cpm.

Avantageusement, les moyens discriminateurs opèrent ladite discrimination additionnelle entre tachycardie ventriculaire et fibrillation ventriculaire par analyse de la stabilité du rythme ventriculaire, notamment une analyse statistique des intervalles RR comprenant l'établissement d'un histogramme des intervalles RR, la recherche d'un pic de stabilité et l'évaluation de la proportion d'intervalles inclus dans ce pic de stabilité.

Après ladite discrimination additionnelle, les moyens discriminateurs peuvent en particulier commander l'application d'une thérapie de choc lorsque le rythme ventriculaire est reconnu instable, et commander l'application d'une stimulation antitachycardique lorsque ce rythme est reconnu stable.

On va maintenant décrire plus en détail la manière dont l'invention est mise en oeuvre, en référence aux dessins annexés.
La figure 1, précitée, illustre les différentes plages de fréquence prises en compte par les algorithmes de discrimination de l'art antérieur, avec les diagnostics et actions correspondants.
La figure 2 illustre les différentes plages de fréquence prises en compte par l'algorithme de discrimination selon l'invention, avec les diagnostics et actions correspondants.

La présente invention peut être mise en oeuvre à partir de l'algorithme déjà connu et décrit dans les EP-A-0 626 182 et EP-A-0 838 235 précités, et qui est utilisé par les modèles de défibrillateurs DEFENDER et ALTO d'ELA Médical pour opérer la détection et la classification des différentes tachyarythmies en fonction de divers critères.

Cet algorithme permet en particulier de détecter et confirmer la survenue de TV par une analyse du rythme cardiaque, cette analyse étant mise en oeuvre dès que la fréquence ventriculaire du rythme recueilli dépasse une fréquence programmée ("fréquence de détection de TV" ou "seuil TDI"). II est en particulier possible de discriminer, entre divers troubles, ceux qui autorisent l'application d'une thérapie antitachycardique (TV lentes ou rapides avérées, FV), et, d'autre part, ceux d'origine non ventriculaire pour lesquels toute thérapie de ce type doit être inhibée : tachycardies supra-ventriculaires ("TSV"), tachycardies sinusales ("TS") et troubles analogues. On pourra se référer aux brevets précités pour de plus amples détails.

La figure 2 illustre les différents cas pris en compte par l'algorithme de discrimination selon l'invention.

Le point de départ de l'invention consiste à placer le seuil FDI à un niveau relativement élevé (typiquement 240 cpm) et à créer en dessous de ce seuil une zone de détection particulière pour la plage de fréquences (typiquement 200 à 240 cpm) où l'algorithme est susceptible de rencontrer aussi bien des FV que des TV (que l'on appellera alors "TV supra-rapides"), afin d'opérer dans cette nouvelle zone une discrimination spécifique, beaucoup plus rapide, basée sur des critères différents de ceux d'une analyse des TV telle qu'elle était opérée auparavant.

Le nouveau seuil correspondant, désigné "seuil F/TDI" est établi à une fréquence inférieure à celle du seuil FDI, par exemple un seuil F/TDI de 200 cpm pour un seuil FDI de 240 cpm.

L'algorithme compare aux différents seuils la valeur de la fréquence du rythme ventriculaire recueilli :
- fréquence inférieure au seuil TDI : rythme considéré comme physiologique, ne justifiant pas l'application d'une thérapie ;
- fréquence comprise entre le seuil TDI et le seuil F/TDI : suspicion de TV, lente ou rapide, et mise en oeuvre d'une analyse plus approfondie du rythme selon les critères de discrimination connus, par exemple ceux décrits dans les brevets précités ;
- fréquence supérieure au seuil FDI : le rythme est considéré d'emblée comme révélateur d'une FV et un choc est appliqué sans délai ;
- fréquence comprise entre le seuil F/TDI et le seuil FDI : analyse supplémentaire, selon l'invention, afin de discriminer entre FV et TV supra rapides.

Cette analyse supplémentaire est avantageusement une analyse de stabilité du rythme ventriculaire (stabilité des intervalles RR), par exemple de la manière décrite dans le EP-A-0 813 888 (ELA Médical), auquel on pourra se reporter pour de plus amples détails.

Essentiellement, l'algorithme analyse un histogramme des intervalles RR mémorisés au cours d'un nombre donné de cycles, par exemple huit cycles. Il détermine un pic central de stabilité et recherche si le pourcentage d'intervalles RR situé à l'intérieur du pic central de stabilité est supérieur ou non à une valeur donnée, par exemple 75 %. Si tel est le cas, l'algorithme conclut à un rythme stable et considère que le trouble détecté est une TV supra-rapide. Dans le cas contraire, il considère qu'il s'agit d'une FV.

Pour pouvoir différencier les thérapies qui seront appliquées (ATP ou choc), en cas de rythme TV l'algorithme incrémente un compteur de persistance de TV rapide, en laissant inchangé un compteur de persistance de FV. En revanche, sur une FV, les compteurs de persistance de FV et de TV sont tous deux incrémentés. Lorsque le compteur de persistance de TV atteint un niveau donné, une thérapie ATP est mise en oeuvre ; si c'est le compteur de persistance de FV qui atteint le premier le seuil de déclenchement de la thérapie, un choc de défibrillation est immédiatement appliqué au patient.

## Revendications

1. Un dispositif médical implantable actif du type défibrillateur, cardioverteur et/ou stimulateur antitachycardique implantable, comprenant :
- des moyens de recueil de l'activité cardiaque ventriculaire et auriculaire,
- des moyens pour appliquer une thérapie antitachycardique par choc de défibrillation ou de cardioversion et/ou par stimulation antitachycardique, et
- des moyens discriminateurs d'arythmies ventriculaires, aptes à analyser le rythme ventriculaire recueilli par rapport à une pluralité de critères incluant la fréquence ventriculaire, cette fréquence étant comparée à une pluralité de seuils comprenant un premier seuil (TDI) de détection des tachycardies ventriculaires et un deuxième seuil (FDI) de détection des fibrillations ventriculaires, ces moyens étant aptes à :
. inhiber l'application des thérapies de choc et de stimulation antitachycardique lorsque la fréquence est inférieure au premier seuil (TDI),
. suspecter une tachycardie ventriculaire et poursuivre l'analyse du rythme sur lesdits critères lorsque la fréquence est comprise entre le premier seuil (TDI) et le deuxième seuil (FDI), et
. commander l'application d'une thérapie de choc lorsque la fréquence est supérieure au deuxième seuil (FDI),
dispositif **caractérisé en ce que** les moyens discriminateurs sont également aptes à comparer la fréquence à un troisième seuil (F/TDI) compris entre le premier seuil (TDI) et le deuxième seuil (FDI), et à :
. suspecter une tachycardie ventriculaire et poursuivre l'analyse du rythme sur lesdits critères seulement lorsque la fréquence est comprise entre le premier seuil (TDI) et le troisième seuil (F/TDI), et
. opérer une discrimination additionnelle entre tachycardie ventriculaire et fibrillation ventriculaire lorsque la fréquence est comprise entre le troisième seuil (F/TDI) et le deuxième seuil (FDI).

2. Le dispositif de la revendication 1 dans lequel le troisième seuil (F/TDI) est compris entre 190 et 210 cpm, pour un deuxième seuil (FDI) compris entre 230 et 250 cpm.

3. Le dispositif de la revendication 1 dans lequel l'intervalle entre troisième seuil (F/TDI) et deuxième seuil (FDI) est compris entre 20 et 50 cpm.

4. Le dispositif de la revendication 1 dans lequel les moyens discriminateurs opèrent ladite discrimination additionnelle entre tachycardie ventriculaire et fibrillation ventriculaire par analyse de la stabilité du rythme ventriculaire.

5. Le dispositif de la revendication 4 dans lequel les moyens discriminateurs sont aptes, après ladite discrimination additionnelle, à :
. commander l'application d'une thérapie de choc lorsque le rythme ventriculaire est reconnu instable, et
. commander l'application d'une stimulation antitachycardique lorsque le rythme ventriculaire est reconnu stable.

6. Le dispositif de la revendication 4 dans lequel ladite analyse de stabilité du rythme ventriculaire est une analyse statistique des intervalles RR.

7. Le dispositif de la revendication 6 dans lequel ladite analyse statistique des intervalles RR comprend l'établissement d'un histogramme des intervalles RR, la recherche d'un pic de stabilité et l'évaluation de la proportion d'intervalles inclus dans ce pic de stabilité.

## Claims

1. An active implantable medical device of the implantable defibrillator, cardioverter and/or antitachycardia pacemaker type comprising:
- means for detecting ventricular and atrial cardiac activity,
- means for delivering an antitachycardia therapy by means of a defibrillation shock, a cardioversion shock, and/or an antitachycardia stimulation, and
- means for discriminating ventricular arrhythmias, being able to analyse the detected ventricular rhythm with respect to a plurality of criteria including the ventricular frequency, said frequency being compared to a plurality of thresholds comprising a first threshold (TDI) of detection of ventricular tachycardias and a second threshold (FDI) of detection of ventricular fibrillations, said means being able to:
■ inhibit the application of said shock or antitachycardia stimulation therapies when the frequency is lower than said first threshold (TDI),
■ suspect a ventricular tachycardia and continue the analysis of the rhythm with respect to said criteria when the frequency is between the first threshold (TDI) and the second threshold (FDI), and
■ command the application of a shock therapy when the frequency is higher than said second threshold (FDI),
device **characterised in that** the discriminating means is equally able to compare the frequency to a third threshold (F/TDI) between the first threshold (TDI) and the second threshold (FDI), and to:
■ suspect a ventricular tachycardia and continue the analysis of the rhythm with respect to said criteria only when the frequency is between the first threshold (TDI) and the third threshold (F/TDI), and
■ perform an additional discrimination between a ventricular tachycardia and a ventricular fibrillation when the frequency is between the third threshold (F/TDI) and the second threshold (FDI).

2. The device of claim 1, wherein the third threshold (F/TDI) is between 190 and 210 bpm, with a second threshold (FDI) between 230 and 250 bpm.

3. The device of claim 1, wherein the interval between the third threshold (F/TDI) and the second threshold (FDI) is between 20 and 50 bpm.

4. The device of claim 1, wherein the discriminating means performs said additional discrimination between a ventricular tachycardia and a ventricular fibrillation by analysing the stability of the ventricular rhythm.

5. The device of claim 4, wherein the discriminating means is able to, after said additional discrimination:
■ command the application of a shock therapy when the ventricular rhythm is determined to be unstable, and
■ command the application of an antitachycardia stimulation when the ventricular rhythm is determined to be stable.

6. The device of the claim 4, wherein the stability analysis of the ventricular rhythm is a statistical analysis of RR-intervals.

7. The device of claim 6, wherein said statistical analysis of RR-intervals comprises establishing a histogram of the RR-intervals, searching for a peak of stability, and evaluating the proportion of intervals included in said peak of stability.

## Patentansprüche

1. Aktive implantierbare medizinische Vorrichtung vom Typ eines implantierbaren Defibrillators, Kardioverters und/oder antitachykardischen Stimulators, mit:
- Mitteln zur Aufnahme der Herzkammer- und Herzvorhofaktivität,
- Mitteln zur Anwendung einer antitachykardischen Therapie durch einen Defibrillations- oder Kardioversionsschock und/oder durch eine antitachykardische Stimulation, und
- Mitteln zur Unterscheidung von Herzkammer-Arrhythmien, die dazu geeignet sind, den aufgenommenen Herzkammerrhythmus bezüglich einer Mehrzahl von Kriterien umfassend die Herzkammerfrequenz zu analysieren, wobei diese Frequenz mit einer Mehrzahl Schwellen verglichen wird, die eine erste Schwelle (TDI) der Erkennung der Herzkammer-Tachykardien und eine zweite Schwelle (FDI) der Erkennung des Herzkammerflimmerns umfassen, wobei diese Mittel dazu geeignet sind:
■ die Anwendung der Schocktherapien und der antitachykardischen Stimulationstherapien zu hemmen, wenn die Frequenz geringer als die erste Schwelle (TDI) ist,
■ eine Herzkammer-Tachykardie zu vermuten und die Analyse des Rhythmus anhand der besagten Kriterien fortzuführen, wenn die Frequenz zwischen der ersten Schwelle (TDI) und der zweiten Schwelle (FDI) liegt, und
■ die Anwendung einer Schocktherapie zu befehligen, wenn die Frequenz höher als die zweite Schwelle (FDI) ist,
Vorrichtung, die **dadurch gekennzeichnet ist, dass** die Unterscheidungsmittel ebenfalls dazu geeignet sind, die Frequenz mit einer dritten Schwelle (F/TDI) zu vergleichen, die zwischen der ersten Schwelle (TDI) und der zweiten Schwelle (FDI) liegt, und dazu:
■ eine Herzkammer-Tachykardie zu vermuten und die Analyse des Rhythmus anhand der besagten Kriterien lediglich dann fortzuführen, wenn die Frequenz zwischen der ersten Schwelle (TDI) und der dritten Schwelle (F/TDI) liegt, und
■ eine zusätzliche Unterscheidung zwischen einer Herzkammer-Tachykardie und einem Herzkammerflimmem vorzunehmen, wenn die Frequenz zwischen der dritten Schwelle (F/TDI) und der zweiten Schwelle (FDI) liegt.

2. Vorrichtung nach Anspruch 1, in welcher die dritte Schwelle (F/TDI) zwischen 190 und 210 cpm liegt, bei einer zweiten Schwelle (FDI) zwischen 230 und 250 cpm.

3. Vorrichtung nach Anspruch 1, in welcher der Intervall zwischen der dritten Schwelle (F/TDI) und der zweiten Schwelle (FDI) zwischen 20 und 50 cpm liegt.

4. Vorrichtung nach Anspruch 1, in welcher die Unterscheidungsmittel die zusätzliche Unterscheidung zwischen einer Herzkammer-Tachykardie und einem Herzkammerflimmern durch Analyse der Stabilität des Herzkammerrhythmus vomehmen.

5. Vorrichtung nach Anspruch 4, in welcher die Unterscheidungsmittel geeignet sind, nach der zusätzlichen Unterscheidung:
■ die Anwendung einer Schocktherapie zu befehligen, wenn der Herzkammerrhythmus als instabil erkannt wird, und
■ die Anwendung einer antitachykardischen Stimulation zu befehligen, wenn der Herzkammerrhythmus als stabil erkannt wird.

6. Vorrichtung nach Anspruch 4, in welcher die Analyse der Stabilität des Herzkammerrhythmus eine statistische Analyse der RR-Intervalle ist.

7. Vorrichtung nach Anspruch 6, in welcher die statistische Analyse der RR-Intervalle die Erstellung eines Histogramms der RR-Intervalle, die Suche nach einer Stabilitätsspitze und die Ermittlung des Anteils an in dieser Stabilitätsspitze enthaltenen Intervallen umfasst.
